# EUROPÄISCHE PATENTANMELDUNG

(11) **EP 0 893 124 A1**
(43) Veröffentlichungstag der Anmeldung: **27.01.1999**
(21) Anmeldenummer: 97112715.4
(22) Anmeldetag: 24.07.1997
(51) Int. Cl.: A61K 39/42, A61K 31/70, A61K 39/395

(54) **Pharmzeutische Kombinationspräparate enthaltend humane monoklonale Antikörper zur Behandlung der chronischen Hepatitis B und eine Virostatisch wirksame Substanz**

(71) Anmelder: BOEHRINGER MANNHEIM GMBH, 68298 Mannheim (DE)
(72) Erfinder: Baumann, Matthias, Dr., 68165 Mannheim (DE); Beck, Walter, Dr., Dipl.-Chem., 68259 Mannheim (DE); Marschner, Jens-Peter, Dr., 64546 Mörfelden-Walldorf (DE)

(57) **Zusammenfassung**

Gegenstand der vorliegenden Erfindung sind pharmazeutische Kombinationspräparate mit synergistischer Wirkung enthaltend humane monoklonale Antikörper zur Behandlung der chronischen Hepatitis B und eine virostatisch wirksame Substanz. Die Präparate können in getrennten Verpackungseinheiten oder auch in einer gemeinsamen Verpackungeinheit vorliegen. Die Präparate eignen sich insbesondere zur Behandlung der chronischen Hepatitis B. Die kombinierte Verabreichung des humanen monoklonalen Antikörpers verstärkt denn virostatischen Effekt der Einzelpräparate.

## Beschreibung

Die vorliegende Erfindung betrifft pharmazeutische Kombinationspräparate enthaltend humane monoklonale Antikörper zur Behandlung der chronischen Hepatitis B und ein virostatisch wirksames Mittel. Die Kombinationspräparate werden insbesondere zur Verstärkung des virostatischen Effektes von Wirkstoffen mit antiviraler Wirkung eingesetzt.

Das Hepatitis B Virus (HBV) verursacht in Europa und USA etwa 5 - 10% der Fälle mit chronischer Lebererkrankung und Lebercirrhose, die in vielen Fallen zu einer Lebertransplantation führen. In asiatischen und afrikanischen Ländern ist das HBV mehr verbreitet und eine der Hauptursachen für tödliche Lebererkrankungen und hepatozellulärem Carcinom (HCC). Durch den Einsatz von Interferon (IFNα) wurden deutliche Fortschritte in der Behandlung der chronischen Hepatitis B (CHB) erzielt. IFNα inhibiert die virale Replikation und moduliert das Immunsystem. Jedoch ist die Behandlung nur bei etwa 30 - 40 % der Patienten erfolgreich. Hierbei ist jedoch zu beachten, daß dieser Wert nur geringfügig über dem Wert von 20 - 25 % der Patienten liegt, bei denen eine spontane Serokonversion ohne die Verabreichung von Medikamenten zu beobachten ist. Oft treten auch Nebenwirkungen (grippeähnliche Symptome, Knochenmarkdepression) auf, die den Einsatz von IFNα beschränken. Die Gründe für den relativ hohen Anteil von 60 - 70 % der Patienten, die auf die IFNα-Therpaie nicht ansprechen (sog. 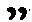 Non-Responder") sind unbekannt. In der Regel werden als 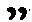 Non-Responder" solche Patienten definiert, die mit IFNα für eine Dauer von mindestens vier Wochen mit einer kumulativen Dosis von mindestens 240 MU behandelt wurden, ohne daß hierbei ein therapeutischer Behandlungserfolg zu verzeichnen war, der durch einen Verlust an HBV-DNA und HBeAg erkennbar ist.

Eine zweite therapeutische Möglichkeit besteht in der Verabreichung von Nukleosid-Analoga mit antiviraler Wirkung (z.B. Lamivudin, Famciclovir, ARA-MP [=Vidarabinmonophosphat]). Diese Nukleosid-Analoga werden in die virale DNA oder RNA eingebaut und führen durch Kettenabbruch oder Blockade von Bindungsstellen auf den DNA- oder RNA-Strängen zum Abbruch der viralen Nukleinsäure-Synthese. Über die Hemmung der Virusreplikation kommt es dann sekundär zur Reduktion der entzündlichen Aktivität in der Leber.

Zur Behandlung der CHB wurden in der Literatur auch der therapeutische Einsatz von monoklonalen oder polyklonalen Antikörpern beschrieben (Lever et al., Lancet, 1990, 335: 1529). Ferner ist auch der Einsatz eines humanen monoklonalen Antikörpers mit der Bezeichnung OST-577 (Oestberg L, Pursch E, Hybridoma 1983; 2(4): 361-367) bekannt. Weitere Ansätze bei der Therapie sind Thymosin und Interleukin 12 (IL 12).

Bisher war jedoch keine der bekannten Therapiemöglichkeiten zur Behandlung der chronischen Hepatitis B allein in der Lage, eine zufriedenstellende oder wirksame Heilung der Patienten herbeizuführen.

Überraschenderweise wurde nun gefunden, daß die Behandlung der CHB und die Inhibierung der Virusreplikation dadurch wesentlich verbessert werden kann, indem eine Kombination von humanen monoklonalen Antikörpern, die gegen das Oberflächenantigen des HBV gerichtet sind, mit antiviralen bzw. virostatisch wirksamen Substanzen verabreicht wird. Insbesondere führt die kombinierte Gabe von humanen monoklonalen Antikörpern zu einer deutlichen Verstärkung der antiviralen Wirkung der Virostatica. Im Falle der IFNα-Therapie konnte die Anzahl der Patienten, die auf die IFNα-Gabe nicht ansprechen ( 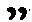 Non-Responser") deutlich erniedrigt werden.

Im Sinne der vorliegenden Erfindung kommen als humane monoklonale Antikörper insbesondere die Antikörper der IgG₁-Unterklasse in Frage, die die a-Determinante des Hepatitis B Virus Oberflächenantigens (HBsAg) erkennen. Derartige Antikörper sind beispielsweise OST-577 oder Tuvirumab (CAS Registry No. 138660-97-6). Diese Antikörper können erhalten werden durch Zellfusion ausgehend von einer 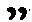 Trioma"-Zellinie (SP2 Maus Myeloma Zeilen x humane periphere Blutlymphozyten (PBL) eines nicht-immunisierten Donors x PBL eines Hepatitis B vakzinierten Donors). Tuvirumab bindet Virionen und Filamente, die als freie Partikel im Blut zirkulieren und an der Oberfläche von Zeilen präsentiert werden.

Virostatisch wirksame Mittel (Virostatica) oder antivirale Substanzen im Sinne der vorliegenden Erfindung sind IFNα oder Nukleosid-Analoga. Als Nukleosid-Analoga kommen solche Derivate von Nukleosiden oder Nukleotiden in Frage, die die Replikation der viralen DNA oder RNA inhibieren. Derartige Nukleosid-Analoga sind beispielsweise Lamivudin (3TC, (-)-3'-Thiacytidin), Ganciclovir, Famciclovir, Adeninarabinosid-monophosphat (ARA-MP), Zidovudin, Didesoxyinosin, Dideoxycytidin, Ribavirin oder Fialuridin.

Durch die erfindungsgemäßen Kombinationspräparate werden die Wirkungen der Einzelpräparate bei der Behandlung der CHB auf synergistische Weise verstärkt. Insbesondere wird die Entstehung von resistenten HBV reduziert. Ferner wird der virostatische Effekt der Einzelpräparate deutlich verstärkt. Im Falle der Nukleosid-Analoga ist es überraschenderweise möglich, die Dauer der Behandlung deutlich zu verkürzen, wodurch auf die Langzeittherapie mit den bekannten Nebenwirkungsprofilen weitgehend verzichtet werden kann.

Im Sinne der vorliegenden Erfindung sollen unter dem Begriff "Kombinationspräparate" nicht nur solche Arzneimittelpackungen verstanden werden, bei denen das Präparat enthaltend den humanen monoklonalen Antikörper zur Behandlung der CHB und das virostatisch wirksame Mittel in einer verkaufsfertigen Verpackungseinheit nebeneinander konfektioniert vorliegen (sogenannte Kombinationspackung), sondern auch solche Arzneimittelpackungen, die entweder eine therapeutisch wirksame Menge des Antikörper-Präparates oder eine geeignete Menge des virostatisch wirksamen Mittels in Form der jeweiligen Einzelpräparate enthalten, wobei die Einzelpräparate hinsichtlich der Menge der Inhaltsstoffe derart konfektioniert sind, daß sie im Sinne der Erfindung für die kombinierte Gabe mit dem jeweils anderen Präparat verabreicht werden können. In diesen Fällen werden den Präparaten in der Regel von den Pharma-Herstellern oder den Arzneimittel-Importeuren ein in vielen Ländern gesetzlich vorgeschriebener Beipackzettel für Arzneimittel beigelegt, in dem Anweisungen oder Informationen über die kombinierte Gabe der Einzelpräparate enthalten sind.

Die Dosis des Antikörpers beträgt 10 - 200 mg, vorzugsweise bis zu 100 mg je Einzeldarreichungsform. Bevorzugt im Falle von Tuviromab kommen Präparate mit einem Wirkstoffgehalt von 20 mg, 40 mg oder 80 mg in Frage. Die Dosierung der virostatisch wirksamen Mitteln richtet sich nach den für diese Wirkstoffe empfohlenen Dosierungen.

Die Behandlung mit dem Kombinationspräparat erfolgt täglich, bevorzugt ein- bis fünfmal wöchentlich, wobei die Präparate gleichzeitig oder zeitlich nacheinander verabreicht werden können. Die Festlegung der individuellen Dosis erfolgt derart, daß folgende Zielkriterien erreicht bzw. erfaßt werden: a) Neutralisieren des HBsAg durch die Verabreichung der erforderlichen Menge des monoklonalen Antikörpers und b) Erreichen der HBV-Negativität durch die antiviralen Substanzen. Hinsichtlich des Zielkriteriums a) kann zunächst eine individualisierte Dosierungsform angestrebt werden, da die Ausgangslage, d.h. der sogenannte 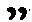 viral load" für die Patienten sehr unterschiedlich sein kann. Zur Erreichung der HBV-Negativität nach b) werden gegebenenfalls in einer ersten Behandlungsphase zunächst die zugelassenen und akzeptierten Dosisbereiche der antiviralen Substanzen verabreicht, während in der Hauptbehandlungsphase eine Dosisanpassung an den durch die Kombination reduzierten Bedarf der antiviralen Substanz durchgeführt wird.

Bei der Anwendung der Kombinationspräparate ist es möglich, die Präparate in einer sogenannten fixen Kombination, d.h. in einer einzigen pharmazeutischen Formulierung zu verabreichen, in der beide Wirkstoffe enthalten sind. Dies können z.B. Injektionslösungen, Infusionslösungen oder Lyophilisate sein, die beispielsweise in Ampullen abgefüllt sind. Die fixe Kombination der beiden Wirkstoffe in Form eines Lyophilisates hat den Vorteil der einfachen und sicheren Handhabung. Das Lyophilisat wird in der Ampulle durch Zugabe pharmazeutisch üblicher Injektionsmedien gelöst und intravenös appliziert.

Es ist auch möglich, das Präparat enthaltend den humanen monoklonalen Antikörper und die virostatisch wirksamen Mittel in Form von getrennten pharmazeutischen Formulierungen zur Verfügung zu steilen. In der Regel erfolgt dies in Form einer einzigen Verpackungseinheit, die zwei Behältnisse umfaßt, wobei das erste Behältnis eine das Antikörper-Präparat enthaltende Darreichungsform (Lyophilisat, Injektions- oder Infusionslösung) ist, und das zweite Behältnis eine geeignete Darreichungsform für die virostatisch wirksamen Mittel darsteilt. Die Verpackungseinheiten können auch mehrere Einzeldosierungspräparate des Antikörper-Präparates oder des virostatisch wirksamen Mittels enthalten, so daß beispielsweise eine Verpackungseinheit die für einen bestimmten Zeitraum (z.B. für die wöchentliche Dosierung) erforderliche Anzahl von Einzeldarreichungsformen beinhaltet.

Diese freie Kombination, die in einer einzigen Verpackungseinheit (Arzneimittelpackung) zur Verfügung gestellt werden kann, hat den Vorteil, daß jedem zu behandelnden Patienten eine bestimmte individuelle Menge des Antikörper-Präparates und des virostatisch wirksamen Mittels zugeordnet werden kann. Derartige Kombinationspräparate bieten außerdem den Vorteil der größeren Sicherheit für den Therapieerfolg, da jeweils die optimal abgestimmte Menge der Einzelpräparate festgelegt ist, und eine Verwechslung mit sonst im Handel erhältlichen Einzelpräparten, die in unterschiedlichen Dosierungen angeboten werden, weitgehend ausgeschlossen werden kann. Zudem ist zu berücksichtigen, daß in verschiedenen Ländern oft Arzneimittelpräparate mit unterschiedlichen Dosierungen aufgrund der nationalen Erfordernisse im Handel sind, und somit eine erhöhte Verwechslungsgefahr mit variierenden Mengenverhältnisse der Einzelwirkstoffe besteht. Die erfindungsgemäßen Kombinationspräparate minimieren ferner das Risiko einer versehentlich zu hohen Dosierung der Einzelpräparate, falls eine Reduzierung des virostatisch wirksamen Mittels in Kombination mit dem Antikörper angezeigt ist. Durch die erfindungsgemäßen Kombinationspräparate wird eine sichere Therpie und einfache Handhabung durch das behandelnde Personal oder im Rahmen der durch den Patienten vorgenommenen Selbstmedikation sichergestellt. Es ist ferner möglich, einen Wirkstoff als Injektionslösung und den anderen Wirkstoff als Darreichungsform zur oralen Verabreichung zur Verfügung zu stellen.

Für den Fall, daß der Antikörper als Lyophilisat zur Verfügung gestellt wird, enthalten die Arzneimittelpackungen (Kombinationspackungen) die entsprechende Menge des Präparates in Glasampullen. Das virostatisch wirksame Mittel kann in fester Form (Tablette, Pulver, Granulat, Lyophilisat, etc.) oder auch in flüssiger Form in einem getrennten Behältnis vorliegen. Ferner enthält die Kombinationspackung vorzugsweise eine Rekonstitutionslösung, um entweder das Wirkstofflyophilisat allein oder auch zusammen mit dem virostatisch wirksamen Mittel aufzulösen. Liegt das virostatisch wirksamen Mittel als gebrauchsfertige Lösung vor, kann die Lösung zusammen mit der Antikörper-Lösung gemischt werden, falls die gemeinsame Applikation von Antikörper und dem virostatisch wirksamen Mittel erfolgen soll.

Kombinationspräparate im Sinne der vorliegenden Erfindung sind auch solche Verpackungseinheiten, die auf eine optimale wöchentlich zu verabreichende Menge des Antikörper-Präparates und des virostatisch wirksamen Mittel abgestellt sind. Vorteilhaft werden wöchentlich 10 - 700 mg des Antikörper-Präparates verabreicht. Diese Gesamtdosis kann in mehreren Teildosierungen für die tägliche Gabe (d.h. 7 mal pro Woche) oder für die Verabreichung von 1 - 6 Teilmengen pro Woche aufgeteilt sein. Die wöchentlich zu verabreichende Menge des Virostatikums kann gegebenenfalls in einer der wöchentlichen Gesamtdosis entsprechenden Menge oder auch in mehreren Teilmengen für eine mehrmalige Gabe pro Woche zusammen mit dem Antikörper-Präparat aufgeteilt sein.

Eine weitere Möglichkeit im Sinne der vorliegenden Erfindung besteht darin, jeweils einzelne Darreichungsformen des Antikörper-Präparates oder des virostatisch wirksamen Mittels als unabhängige Arzneimittel zur Verfügung zu stellen, wobei die Einzelpräparate derart konfektioniert sind, daß sie die erforderlichen Menge der Einzelsubstanzen für die erfindungsgemäße Kombination enthalten. In der Regel enthalten die Arzneimittelpackungen die vorgeschriebenen Beipackzettel, in denen ein entsprechender Hinweis für die kombinierte Gabe des Antikörpers bzw. des virostatisch wirksamen Mittels in der erforderlichen Menge enthalten ist. Ein entsprechender Hinweis kann auch als Verpackungsaufdruck auf der Arzneimittelpackung (Sekundärpackmittel) oder dem Primärpackmittel (Ampulle, Blisterstreifen, etc.) enthalten sein.

Die Herstellung der pharmazeutischen Darreichungsformen erfolgt nach üblichen, in der galenischen Technik bekannten Verfahren mit pharmazeutisch üblichen Hilfsstoffen.

Bei der Durchführung der Kombinationstherapie mit dem erfindungsgemäßen Kombinationspräparat kann auf sehr einfache Art und Weise über die wöchentliche maximale Dosierung entschieden werden, indem die bekannten diagnostischen Parameter zum Nachweis der HBV-Infektion bestimmt werden.

### Beispiel 1

Patienten werden mit einer wöchentlichen Dosis von Tuvirumab und einer wöchentlichen Dosis von IFNα behandelt. Die beiden Präparate werden jeweils um 24 Stunden versetzt verabreicht. Durch Bestimmung geeigneter diagnostischen Parameter (wie z.B. der sog. 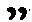 viral load") wird der Behandlungserfolg der Patienten erfaßt.

### Beispiel 2

Patienten werden mit einer Tagesdosis von Tuvirumab und einer Tagesdosis von Lamivudin behandelt. Die beiden Präparate werden jeweils am gleichen Tag verabreicht. Durch Bestimmung geeigneter diagnostischen Parameter wird der Behandlungserfolg der Patienten erfaßt.

## Patentansprüche

1. Pharmazeutisches Kombinationspräparat umfassend einen humanen monoklonalen Antikörper zur Behandlung der chronischen Hepatitis B und eine virostatisch wirksame Substanz.

2. Kombinationspräparat nach Anspruch 1, dadurch gekennzeichnet, daß der humane monoklonale Antikörper gegen das Hepatitis B Oberflächenantigen (HBsAg) gerichtet ist.

3. Kombinationspräparat nach Anspruch 2, dadurch gekennzeichnet, daß der monoklonale Antikörper Tuvirumab ist.

4. Kombinationspräparat nach einem der Ansprüche 1 - 3, dadurch gekennzeichnet, daß die virostatisch wirksame Substanz IFNα ist.

5. Kombinationspräparat nach einem der Ansprüche 1- 3, dadurch gekennzeichnet, daß die virostatisch wirksame Substanz ein Nukleosidanalogon ist.

6. Kombinationspräparat nach einem der Ansprüche 1- 3, dadurch gekennzeichnet, daß die virostatisch wirksame Substanz Lamivudin ist.

7. Verfahren zur Herstellung eines Kombinationspräparates nach einem der Ansprüche 1 - 6, dadurch gekennzeichnet, daß man humane monoklonale Antikörper zur Behandlung der chronischen Hepatitis B in Form einer geeigneten Einzeldarreichungsform und eine virostatisch wirksame Substanz entweder zusammen oder getrennt voneinander mit pharmazeutischen üblichen Träger- oder Hilfsstoffen formuliert und die betreffenden Präparate in Form von Kombinationspräparaten zur Verfügung stellt.

8. Verwendung eines humanen monoklonalen Antikörpers, der gegen das Hepatitis B Oberflächenantigen (HBsAg) gerichtet ist, zur Herstellung von Arzneimitteln zur Verstärkung der antiviralen Wirkung von virostatisch wirksamen Arzneimitteln.

9. Verwendung eines humanen monoklonalen Antikörpers nach Anspruch 9 in Kombination mit IFNα zur Herstellung von Arzneimitteln zur Behandlung der chronischen Hepatitis B bei Patienten, bei denen die Behandlung mit IFNα alleine nicht anspricht.

10. Pharmazeutische Verpackungseinheit umfassend eine therapeutisch wirksame Menge eines humanen monoklonalen Antikörpers und eine virostatisch wirksame Substanz als einheitliche Darreichungsform in einem Behältnis oder als getrennte Darreichungsformen in getrennten Behältnissen.

11. Verpackungseinheit nach Anspruch 10, dadurch gekennzeichnet, daß jeweils der humane monoklonale Antikörper und die virostatisch wirksame Substanz in getrennten Darreichungsformen in Form von Lösungen für Injektions- oder Infusionszwecke vorliegen.
